# EUROPÄISCHE PATENTSCHRIFT

(11) **EP 2 253 351 B1**
(45) Veröffentlichungstag und Bekanntmachung des Hinweises auf die Patenterteilung: **01.06.2011**
(21) Anmeldenummer: 09158904.4
(22) Anmeldetag: 28.04.2009
(51) Int. Cl.: A61M 16/20, A61M 16/00

(54) **Schlitzventil in Kombination mit einem pneumatischen Schaltkreis eines Beatmungsgeräts**
Slit valve in combination with a pneumatic switching circuit of a ventilator device
Soupape à rainure en combinaison avec un circuit pneumatique d'un appareil respiratoire

(43) Veröffentlichungstag der Anmeldung: 24.11.2010
(73) Patentinhaber: Dräger Medical GmbH, 23558 Lübeck (DE)
(72) Erfinder: von Blumenthal, Tilman, 23562, Lübeck (DE); Koulechov, Kirill, Dr., 23669 Timmendorfer Strand (DE); Hansmann, Hans-Ullrich, 23858 Barnitz (DE); Hengstenberg, Andreas Dr., 23858, Reinfeld (DE); Neumann, Andreas, 23826 Klempau (DE); Wall, Torge, 23562, Lübeck (DE); Otto, Andreas, 22941, Bargteheide (DE); Kämer, Markus, 23627 Groß Grönau (DE)
(74) Vertreter: UEXKÜLL & STOLBERG

(56) Entgegenhaltungen:
- EP-A- 0 474 069
- EP-A- 1 459 774
- DE-A1- 4 142 295
- GB-A- 966 137
- US-A1- 2003 189 067

## Beschreibung

ARDS (acute respiratory deficiency syndrome) bezeichnet ein plötzliches Lungenversagen, das durch einen akuten Entzündungsprozess des Lungengewebes entsteht, bei dem die Lunge weitgehend ihre Fähigkeit zum Gasaustausch verliert. Bei ARDS vergrößert sich die Durchlässigkeit der Blutgefäße in den Lungenbläschen, und der Druck in den Gefäßen fällt ab, während er in anderen Teilen des Lungengewebes ansteigt. Dadurch kommt es zu einer lebensbedrohlichen Atemnot und zu einer Unterversorgung des Blutes mit Sauerstoff. Die lebensbedrohliche Sauerstoffarmut des Blutes muss schnellstmöglich mit Hilfe einer maschinellen Unterstützung der Atmung behandelt werden, d.h. künstliche Beatmung mit sauerstoffangereicherter Luft. Nicht-invasive Beatmungsverfahren mit einer bloßen Erhöhung der Sauerstoffkonzentration der zugeführten Atemluft reichen für die Behandlung von ARDS aber oft nicht aus, da beatmete ARDS-Patienten atelektatische (also zusammengefallene) Lungenbereiche aufweisen, die nur mit einem hohen Beatmungsdruck geöffnet (rekrutiert) und für den Gasaustausch nutzbar gemacht werden können. Hierzu muss der Patient allerdings intubiert werden, d.h. dem Patienten wird ein Tubus (Schlauch) durch den Mund oder durch die Nase in die Luftröhre geschoben. Vorzugsweise erfolgt die Beatmung über einen Endotrachealtubus oder über eine Tracheotomiekanüle. Ein Endotrachealtubus besteht normalerweise aus einem dünnen, an beiden Enden geöffneten Schlauch, dessen unteres Ende in die Luftröhre geschoben wird. Kurz oberhalb des unteren Endes befindet sich eine Blockmanschette, die über einen dünnen Schlauch, der an der Seite des Tubus verläuft, aufgeblasen werden kann. Dadurch wird dann die Luftröhre abgedichtet. Am oberen Ende ist der Endotrachealtubus mit einem normierten Verbindungsstück ausgestattet, das den Anschluss an ein Beatmungsgerät ermöglicht. Eine Tracheotomiekanüle wird bei einem Luftröhrenschnitt (Tracheotomie) verwendet. Auch die Tracheotomiekanüle weist einen aufblasbaren "Block" auf, der eine Beatmung ermöglicht und gleichzeitig verhindert, dass Rachensekret nach unten in die Lunge gelangen kann.

Bei der künstlichen Beatmung von ARDS-Patienten wird vorzugsweise am Ende der Expiration ein Restdruck (PEEP - positive endexspiratory pressure) gehalten. Durch die PEEP-Beatmung wird der Druck in den Lungenbläschen erhöht, wodurch die Lungenbläschen gedehnt werden, was zu einer Vergrößerung der Fläche für den Gasaustausch und somit zu einer Verbesserung der Sauerstoffaufnahme führt. Ferner wird die Gefahr des Zusammenfallens der Lungenbläschen beim Ausatmen verringert. Bei ARDS-Patienten beträgt der endexspiratorische Druck bei der PEEP-Beatmung häufig 10 mbar und mehr, um das Rekollabieren der mühsam geöffneten Lungenareale zu vermeiden. In den meisten Fällen ist es erforderlich, einen ARDS-Patienten über mehrere Tage oder sogar Wochen künstlich zu beatmen. Im Verlauf der Beatmung sind allerdings im klinischen Ablauf einige Schritte erforderlich, wie zum Beispiel Absaugung von Flüssigkeiten aus der Lunge, Umlagerung des Patienten, Wechsel des Schlauchsystems, des Filters oder des Beatmungsgeräts. Bei der Durchführung dieser klinisch notwendigen Schritte kann der erforderliche Lungendruck nicht durchgehend aufrechterhalten werden, so dass die geschädigten Lungenareale immer wieder neu rekrutiert werden müssen.

Die US 4,351,328 beschreibt einen Adapter, der zum Anschließen eines Beatmungsgeräts und eines Endotrachealtubus ausgestaltet ist. Der Adapter ist ferner mit einer Öffnung versehen, die mit Hilfe eines Ventils geschlossen ist. Das Ventil ist als Schlitzventil ausgestaltet und kann vom Absaugschlauch durchstoßen werden, wenn dieser von außen in die Öffnung eingeführt wird.

Die US 4,416,273 offenbart einen Verbindungsadapter für einen Endotrachealtubus. Der Adapter verfügt über einen mit einem Lamellenventil versehenen Anschluss, um einen Absaugkatheter von außen in den Tubus einführen zu können.

Die DE 32 04 110 C2 betrifft einen Trachealtubus zur künstlichen Beatmung. Der untere Teil des Trachealtubus ist von einer Ballonmanschette umgeben, die über eine Aufblaskanüle so weit aufgeblasen werden kann, dass sie gegen die Tracheawand anliegt. Im Innenraum des Trachealtubus sind ein mit einem Respirator verbundener Beatmungsschlauch und eine Druckmesskanüle vorgesehen, um den Druckabfall im Beatmungsschlauch bzw. den intratrachealen Druck messen zu können.

Die DE 198 38 370 C1 beschreibt eine Vorrichtung zum Entfernen von Sputum aus einem Luftröhrenkatheter. Die Vorrichtung weist drei Öffnungen auf, wobei eine erste Öffnung mit dem aus der Luftröhre ragenden Ende des Katheters verbunden ist, eine zweite Öffnung mit einem Luftfilter zum Reinigen und Entkeimen der einzuatmenden Luft gekoppelt werden kann, und eine dritte Öffnung mit einem Auffangbeutel für den Abfluss des Sputums verbunden ist. Ein unter Federspannung stehender Kolben zum Verschließen der dritten Öffnung wird durch die Atemluft beim Ein- und Ausatmen gesteuert.

Die DE 41 42 295 C2 betrifft ein Ventil zur Erzeugung eines Steuerdrucks in einem pneumatischen Schaltkreis. Das Ventil hat die Ausgestaltung eines kreisförmigen Verschlusselements und weist Einschnitte auf, so dass acht Kreissegmente entstehen, die um die Umfangslinie des Verschlusselements aufbiegbar sind. Das Ausmaß der Aufbiegung verändert sich abhängig vom Druck des auf einer Seite des Ventils wirkenden Fluids.

Die DE 10 2005 014 650 B3 offenbart ein Anschlussstück mit einem distalen und einem proximalen Ende zum Anschließen eines Trachealtubus und eines Beatmungsgeräts sowie mit einer Abzweigung zum Einführen eines Katheters. In der Abzweigung ist ein Ventil aus einem zumindest bereichsweise elastisch verformbaren Material vorgesehen, das einen Schnabelabschnitt mit einem Schlitz bildet, der beim Einführen des Katheters geöffnet wird.

Geschlossene Absaugungen, wie sie in diesem Dokument gezeigt sind, verhindern lediglich den Druckabfall beim Absaugen. Ein Gerätewechsel und der aus hygienischen Gründen notwendige 48-stündige Wechsel der geschlossenen Absaugung selbst führen nach wie vor zum Lungenkollaps und zu einem anschließenden belastenden Rekrutierungsmanöver.

Die EP 1 459 774 A1 offenbart ein Schlitzventil für eine medizinische Flüssigkeit und eine Gasflusssteuerung in eine Kanüle mit einer Steuerung für das Öffnen und Schließen des Schlitzventils. Hierbei ist das Schlitzventil mit einer Schließmembran ausgeführt, die einen zentral darin ausgebildeten Schlitz enthält.

Die GB 966,137 offenbart in einem Schlitzventil ein zusätzliches Element vorzusehen, welches die durch die Schlitze erzeugten Lamellen in einer Richtung in ihrer Ausbreitung begrenzt.

Keine der vorstehend genannten Schriften beschäftigt sich mit der Beatmung von ARDS-Patienten, und keine dieser Schriften offenbart Beatmungsgeräte bzw. Beatmungseinrichtungen im pneumatischen Schaltkreis zwischen einem Beatmungsgerät und einem Patienten, die ausgestaltet sind, um einen bestimmten Luftdruck in der Lunge des zu beatmenden Patienten auch dann aufrechtzuerhalten, wenn beispielsweise das Beatmungsgerät ausgewechselt wird.

Es ist daher Aufgabe der vorliegenden Erfindung, ein Ventil zur Verwendung im pneumatischen Schaltkreis eines Beatmungsgeräts zur Verfügung zu stellen, mit Hilfe dessen die vorstehend genannten Nachteile überwunden werden. Es ist insbesondere Aufgabe der Erfindung, ein Ventil zur Verwendung in oder an einem mit dem Beatmungsgerät gekoppelten Tubus (z.B. ein Endotrachealtubus oder eine Tracheotomiekanüle) zur Verfügung zu stellen, mit Hilfe dessen ein Druckabfall in der Lunge eines Patienten (insbesondere eines ARDS-Patienten) wirksam verhindert werden kann.

Diese und weitere Aufgaben werden durch ein Schlitzventil mit den Merkmalen des Patentanspruchs 1 gelöst. In den abhängigen Patentansprüchen sind vorteilhafte und bevorzugte Weiterbildungen des erfindungsgemäßen Schlitzventils angegeben.

Ein wesentlicher Vorteil des erfindungsgemäßen Schlitzventils besteht darin, dass das Ventil bidirektional wirkt und abhängig von der Strömungsrichtung bzw. Wirkrichtung eines Fluids bei verschiedenen Schwelldrücken anspricht.

Ein weiterer Vorteil besteht darin, dass das erfindungsgemäße Schlitzventil selbstschließend ist und erst bei Überschreiten eines Schwelldrucks in den geöffneten Zustand übergeht, um das Durchströmen eines Fluids zu ermöglichen. Die Menge des durchströmenden Fluids ist dabei abhängig vom Druck des Fluids. Das erfindungsgemäße Ventil kann von beiden Richtungen (also bidirektional) durchströmt werden, wobei der Schwelldruck, der ein Öffnen des Schlitzventils bewirkt, abhängig von der Strömungsrichtung verschieden ist. Vorzugsweise liegt der Schwelldruck in einer ersten Richtung in einem Bereich zwischen etwa 0 mbar und etwa 5 mbar und in einer zweiten Richtung in einem Bereich zwischen etwa 5 mbar und etwa 15 mbar. Die genaue Höhe dieser Schwellwerte hängt aber vom Anwendungsbereich des Ventils ab und kann beispielsweise auch im Bereich von deutlich über 10 mbar bis zu einigen 100 mbar liegen.

Das erfindungsgemäße Schlitzventil findet vorzugsweise im pneumatischen Schaltkreis eines Beatmungsgeräts Anwendung und ist folglich im Strömungspfad zwischen dem Beatmungsgerät und dem zu beatmenden Patienten vorgesehen, speziell ein ARDS-Patient, der vorzugsweise mit PEEP beatmet wird. Mit Hilfe des erfindungsgemäßen Schlitzventils kann bei Diskonnektion am Endotrachealtubus oder an der Tracheotomiekanüle (wenn beispielsweise das Beatmungsgerät ausgetauscht wird) der Abfall des Drucks in der Lunge des Patienten unter ein vorbestimmtes Druckniveau von beispielsweise zwischen etwa 5 und 15 mbar vermieden werden. Das Ventil ist ferner ausgestaltet, um ein Einatmen zu ermöglichen, ohne dass der Patient hierzu einen großen Ansaugdruck aufbringen muss. Daher liegt der Schwellwert zum Öffnen des Ventils in Einsaugrichtung vorzugsweise bei unter 5 mbar. In Richtung des Ausatmens muss der erforderliche Schwelldruck zum Öffnen des Ventils größer sein und beträgt mehr als 5 mbar, vorzugsweise mehr als 10 mbar oder in Ausnahmefälle mehr als 15 mbar, wobei diese Werte in einer besonders vorteilhaften Ausführung auch abhängig von dem zu beatmenden Patienten, abhängig von der Ausprägung des ARDS und abhängig von anderen Faktoren variieren können. Ferner ist das Ventil ausgestaltet, um das Absaugen von Flüssigkeiten aus der Lunge mit Hilfe einer speziellen Kanüle zu ermöglichen. Das erfindungsgemäße Schlitzventil weist eine hygienische Gestaltung auf, die den Gebrauch über mehr als 1 Woche erlaubt. In einer bevorzugten Ausführung ist das Ventil in spezieller Weise ausgestaltet, um während der normalen Beatmung den Atemstrom nicht zu behindern. Hierzu ist das erfindungsgemäße Schlitzventil mit manuell betätigbaren Einrichtungen versehen, um auf einfache Weise eine Umstellung zwischen verschiedenen Betriebsarten vornehmen zu können.

In der bevorzugten Ausgestaltung ist das erfindungsgemäße Schlitzventil durch eine aus elastischem Kunststoff oder Gummi hergestellte Membran gebildet. Die Membran hat vorzugsweise eine runde Basisform, d.h. eine kreisrunde Umrisslinie. Denkbar sind natürlich auch andere Formen, wie zum Beispiel oval, rechteckig oder quadratisch, die runde Form ist aber aufgrund der Symmetrie bevorzugt. In der Mitte der Membran sind mehrere, sich kreuzende Schlitze vorgesehen, die sich vollständig durch die Membran erstrecken und somit mehrere kreissegmentförmige Lamellen bilden. So werden zum Beispiel durch zwei sich im rechten Winkel kreuzende Schlitze insgesamt vier Lamellen gebildet, durch drei Schlitze werden sechs Lamellen gebildet, etc. Ausgestaltungen mit mehr als sechs Lamellen sind ebenfalls möglich.

Im Ruhezustand (d.h. im Wesentlichen keine Druckdifferenz zischen den gegenüberliegenden Seiten der Membran) sind die Lamellen geschlossen, d.h. die Schlitzflächen benachbarter Lamellen stoßen abdichtend gegeneinander an. Gemäß einer ersten Ausgestaltung liegen die Membranlamellen in ihrer geschlossenen Stellung in einer gewölbten Fläche. Diese gewölbte Fläche kann beispielsweise die Form einer Kuppel bzw. eines Kugelflächensegments haben oder der Außenfläche eines flachen Kegels oder einer flachen Pyramide entsprechen. Unabhängig von der gewählten Ausführung sind die Lamellen so ausgestaltet, dass sie sich bei Aufbringung von Druck (der zum Beispiel über ein Fluid auf die Membranlamellen wirkt) leichter in eine erste Richtung öffnen bzw. verbiegen als in eine zweite Richtung. Folglich ist in einer ersten Strömungsrichtung kein oder nur ein geringer Fluiddruck (erster Schwelldruck) erforderlich, um die Lamellen aus ihrer geschlossenen Position in ihre geöffnete Position zu biegen (d.h. das Ventil zu öffnen), während in der entgegengesetzten zweiten Strömungsrichtung ein größerer Druck (zweiter Schwelldruck) erforderlich ist.

Gemäß einer zweiten Ausgestaltung liegen die Membranlamellen in diesem geschlossenen Zustand in einer im Wesentlichen planaren Ebene und sind ausgestaltet, um bei Druckbeaufschlagung in verschiedenen Richtungen unterschiedliche Schwellwerte zum Öffnen des Ventils bzw. der Lamellen zu realisieren. Dies kann dadurch erreicht werden, dass die Materialstärke der Lamellen in einer axialen Richtung dicker ist als die Materialstärke des ringförmigen Randbereichs bzw. Halterings der Ventilmembran. Dadurch kann bewirkt werden, dass der Kontaktbereich der Schlitzflächen in axialer Richtung relativ groß ist. Gleichzeitig sind aber die sich im Übergang zwischen dem Haltering und den Lamellen befindlichen Schwenklinien der Lamellen gegenüber der axialen Mitte der radial verlaufenden Kontaktbereiche zwischen den Lamellen in axialer Richtung leicht versetzt, so dass in einer axialen Richtung ein größerer Druck aufgebaut werden muss, um die Lamellen aus der geschlossenen Position in die geöffnete Position zu verschwenken bzw. umzuklappen. Alternativ kann die Materialstärke der Lamellen ausgehend von deren Schwenklinien in radialer Richtung auf die Mitte der Ventilmembran zunehmen, wodurch ein ähnlicher Effekt bewirkt wird. Auch ist es möglich, eine ringförmige Nut an einer Seite der Ventilmembran vorzusehen. Durch diese Nut werden die "Filmgelenke" für die Membranlamellen gebildet, wobei aber gleichzeitig durch das Vorsehen der ringförmigen Nut die Schwenklinien der Gelenke in axialer Richtung verlagert werden. Folglich ist auch bei dieser Ausgestaltung der Schwelldruck zum Öffnen des Ventils in einer Richtung größer als in der entgegengesetzten Richtung. Die Höhe der jeweiligen Schwellwerte kann durch die Materialstärke der Membran, die Tiefe der Nut und durch die Elastizität des Membranmaterials bestimmt werden. Ferner ist es möglich, die Membranlamellen an einer Seite direkt an den aneinander anstoßenden Schlitzflächen zwischen benachbarten Lamellen mit sich axial erstreckenden Vorsprüngen zu versehen, um so die Kontaktflächen der Schlitze in axialer Richtung zu vergrößern, wodurch gleichzeitig eine relative axiale Verschiebung der Schwenklinien der Lamellen bewirkt wird.

Das Schlitzventil der vorliegenden Erfindung ist zum Beispiel geeignet, um in den Atemkreis für Überdruckbeatmung (z.B. PEEP) zwischen einem Beatmungsgerät und dem Patienten eingesetzt zu werden. Es sind aber auch andere Anwendungen denkbar, in denen eine ähnliche Ventilfunktion gewünscht ist.

Das erfindungsgemäße Schlitzventil weist vorzugsweise ein im Wesentlichen rohrförmiges Ventilgehäuse mit zwei allgemein gegenüberliegenden Anschlüssen auf und kann in zwei entgegengesetzten Richtungen mit Atemgas durchströmt werden. Wie vorstehend erläutert, weist das erfindungsgemäße Schlitzventil im Strömungskanal zwischen den beiden Anschlüssen eine Schlitzmembran auf, die an ihrem ringförmigen Rand direkt oder indirekt in dem Gehäuse fixiert ist und in ihrer Mitte über durch Einschnitte im Wesentlichen radial getrennte Membranlamellen verfügt. Die Membranlamellen sind gemäß einer bevorzugten Ausführungsform zumindest teilweise so gewölbt, dass bei geschlossenem Ventil die konvexe Seite der Wölbung in Richtung des Patienten zeigt. Beim Ausatmen führt ein mäßiger Überdruck auf der Patientenseite gegen die konvexe Wölbung zunächst zu einer Verpressung der Wölbung entlang der radialen Trennlinien (Schlitze) zwischen den Membranlamellen und damit zu einer Versperrung des Strömungskanals. Erst bei Überschreiten eines vorbestimmten Schwelldrucks von zum Beispiel etwa 10 mbar wird diese Sperrkraft überwunden, die Lamellen werden in Gegenrichtung (also entgegen der Richtung der Wölbung) umgeklappt, und der Strömungskanal wird freigegeben, um von Atemgas durchströmt zu werden. Wenn der Schwelldruck wieder unterschritten wird, klappen die Membranlamellen aufgrund ihrer eigenen Rückstellkräfte in ihre ursprüngliche Wölbung zurück, und der Strömungskanal ist wieder versperrt. Beim Einatmen führt ein Überdruck auf der Gegenseite (d.h. auf der Seite des Beatmungsgeräts) gegen die konkave Unterseite der Wölbung hingegen zu einer sofortigen, nahezu kraftfreien Durchströmung von Fluid in Richtung des Patienten, da nur ein sehr geringer Schwellwert erforderlich ist, um die Lamellen zu öffnen.

Das erfindungsgemäße Ventil kann ferner als ein "Pop-Up" Ventil ausgestaltet sein. Dazu ist zwischen dem inneren kreisförmigen Schlitzbereich und dem äußeren Haltering ein eindrückbarer bzw. faltbarer, ringförmiger Zwischenbereich vorgesehen. Im geschlossenen Ruhezustand des Ventils bzw. in dem Zustand, der vorherrscht, wenn der Patient einatmet und nur ein geringer Schwellwert (Überdruck auf der Seite des Beatmungsgeräts) zum Öffnen der Membranlamellen erforderlich ist, ist dieser Zwischenbereich zusammen- bzw. ineinandergefaltet. Wenn der Patient ausatmet (Überdruck auf der Patientenseite), wird zuerst der zusammengefaltete Zwischenbereich entfaltet. Wenn der Druck weiter ansteigt und den genannten Schwellwert (z.B. 10 mbar oder mehr) übersteigt, werden auch die Membranlamellen umgeklappt. Es hat sich herausgestellt, dass die Membranlamellen bei zusammengefaltetem Zwischenbereich und Überdruck auf der Patientenseite besser gegeneinander verpresst werden. Erst dann, wenn der Zwischenbereich entfaltet ist, erhält der Übergangsabschnitt zwischen den Membranlamellen und dem Zwischenbereich die nötige Flexibilität, um ein leichtes Umklappen der Membranlamellen zu ermöglichen, wenn der patientenseitige Druck beim Ausatmen den vorbestimmten Schwellwert übersteigt. Somit wirkt der faltbare Zwischenbereich als eine Art Sicherungseinrichtung gegen ein frühzeitiges Umklappen der Membranlamellen unterhalb des Schwelldrucks.

Bei einer weiteren vorteilhaften Ausgestaltung der Erfindung ist zusätzlich eine Freigabeeinrichtung vorgesehen, die bei Betätigung die Membranlamellen aus der geschlossenen Position herausklappt, so dass der Strömungskanal im Inneren des Ventils freigegeben ist. Bei einer bevorzugten Ausführung des erfindungsgemäßen Schlitzventils wird das Ventil zwischen einem Endotrachealtubus oder einer Tracheotomiekanüle auf der einen Seite (Patientenseite) und dem Filter, der künstlichen Nase, der geschlossenen Absaugung oder dem Y-Stück auf der anderen Seite (Seite des Beatmungsgeräts) in den Atemkreis eingebracht. Das Ventil hat ein Gehäuse, eine Schlitzmembran mit beispielsweise vier oder sechs radial verlaufenden Schlitzen, wodurch geschnittene Membranlamellen gebildet werden, einen Drehring und einen Offenhalter. Der Offenhalter kann so in den Wirkungsbereich der Schlitzmembran eingeschoben werden, dass die Membranlamellen der Schlitzmembran dauerhaft die Strömungsmitte freigeben und keinen relevanten Strömungswiderstand darstellen. Der Offenhalter verfügt über zwei schräge Haltenasen, die durch das Gehäuse hindurch in eine als schräge Bahn ausgeführte Nut des Drehrings münden. Der Drehring verfügt zusätzlich über eine ringförmige Nut, in die eine Wulst des Gehäuses einrastet. Dadurch ist der Drehring gegen axiale Verlagerung am Gehäuse fixiert. Bei Verdrehen des Drehrings wird der Offenhalter über die zwei schrägen Bahnen axial verschoben und kann so wahlweise in eine Position nahe dem Y-Stück gebracht werden, wo der Offenhalter nicht mit der Membran eingreift und ein Druckabfall in der Lunge des Patienten unterhalb von beispielsweise 10 mbar vermieden wird. In der gegenüberliegenden, patientennahen Position greift der Offenhalter mit den Membranlamellen ein und drückt diese aus der Strömungsmitte heraus, wodurch ein ungehindertes, bidirektionales Durchströmen von Fluid durch das Ventil ermöglicht wird.

Die vorliegende Erfindung wird nun anhand eines Beispiels unter Bezugnahme auf die Zeichnungen beschrieben.
Figur 1a zeigt eine schematische Draufsicht auf eine exemplarische Schlitzmembran gemäß der Erfindung.
Figur 1b zeigt eine Querschnittsansicht durch Linie A-A aus Figur 1a, in der der gewölbte Lamellenbereich zu sehen ist. Die Pfeile stellen die Druckrichtung dar, bei der ein geringer Schwellwert zum Öffnen des Ventils erforderlich ist.
Figur 1c zeigt eine Querschnittsansicht durch Linie A-A aus Figur 1a, in der eine alternative Ausgestaltung zu Figur 1b mit einer variierenden Materialstärke der Membranlamellen zu sehen ist.
Figur 1d zeigt eine Querschnittsansicht durch Linie A-A aus Figur 1a, in der eine weitere alternative Ausgestaltung zu Figur 1c mit einer konstanten Materialstärke der Membranlamellen zu sehen ist, wobei die Materialstärke des Halterings deutlich geringer ist als die der Membranlamellen.
Figur 1e zeigt eine Querschnittsansicht durch Linie A-A aus Figur 1a, in der noch eine alternative Ausgestaltung zu Figur 1d mit einer konstanten Materialstärke der gesamten Membran zu sehen ist, wobei zwischen dem Haltering und dem mittleren Lamellenbereich eine ringförmige Nut vorgesehen ist.
Figur 2a zeigt eine Ausgestaltung der erfindungsgemäßen Schlitzmembran aus Figur 1b im geschlossenen Zustand.
Figur 2b zeigt die Schlitzmembran aus Figur 2a in einem geöffneten Zustand, wobei der Druck in Richtung des Einatmens wirkt und nur ein sehr geringer Druck erforderlich ist, um die Membranlamellen nach unten zu drücken.
Figur 2c zeigt die Schlitzmembran aus Figur 2a in einem geöffneten Zustand, wobei der Druck in Richtung des Ausatmens wirkt und ein recht hoher Druck-Schwellwert erforderlich ist, um die Membranlamellen nach oben umzuklappen.
Figur 3a zeigt eine weitere bevorzugte Ausgestaltung des Schlitzventils aus Figuren 1 und 2 im geschlossenen Zustand.
Figur 3b zeigt das Schlitzventil aus Figur 3a kurz vor Erreichen des geöffneten Zustands in Richtung des Ausatmens.
Figur 3c zeigt eine Abwandlung des Schlitzventils aus Figur 3a im geschlossenen Zustand.
Figur 3d zeigt das Schlitzventil aus Figur 3c kurz vor Erreichen des geöffneten Zustands in Richtung des Ausatmens.
Figur 4 zeigt eine weitere Ausführungsform des erfindungsgemäßen Schlitzventils, das mit einer manuell zu betätigenden Freigabeeinrichtung versehen ist.

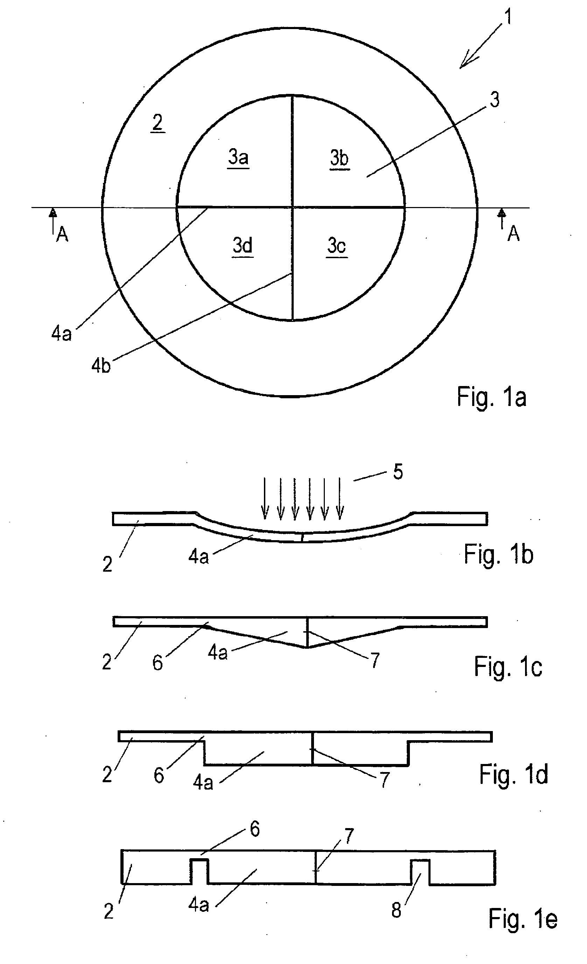

Im Folgenden werden die Figuren detailliert beschreiben. Figur 1 a zeigt eine Draufsicht auf eine exemplarische Schlitzmembran gemäß der Erfindung in ihrer einfachsten Ausgestaltung. Die Schlitzmembran 1 hat im Wesentlichen die Form einer Scheibe und ist aus Gummi, Silikonkautschuk oder einem geeigneten elastischen Kunststoffmaterial hergestellt. Die Schlitzmembran 1 umfasst einen kreisförmigen Lamellenbereich 3 mit Lamellen 3a bis 3d, die durch sich kreuzende Schlitze 4a, 4b gebildet sind, und einen ringförmigen Haltebereich 2, der den Lamellenbereich 3 umgibt und der Befestigung der Schlitzmembran in einem vorzugsweise ringförmigen Ventilgehäuse dient. In Figur 1 a sind zwei sich kreuzende Schlitze gezeigt, wodurch vier kreissegmentförmige Lamellen gebildet werden. Es ist aber auch möglich, drei oder mehr sich kreuzende Schlitze vorzusehen, wodurch sich die Gesamtzahl der Lamellen entsprechend erhöht.

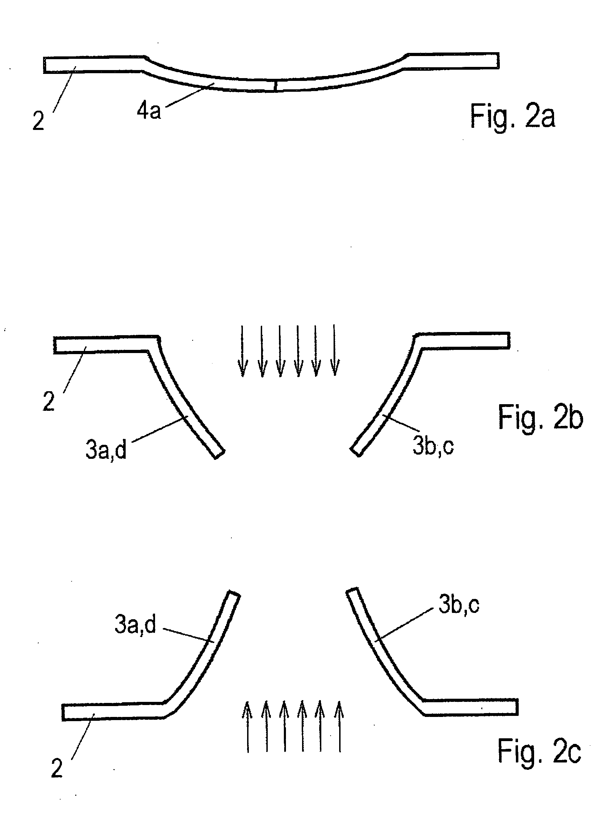

Figur 1b zeigt eine Querschnittsansicht durch Linie A-A aus Figur 1a, in der der gewölbte Lamellenbereich 3 entlang des Schlitzes 4a zu sehen ist. In dieser Ausführung liegen die Membranlamellen 3a-3d in ihrer geschlossenen Position in einer gewölbten Fläche, die im vorliegenden Fall die Form einer Kuppel bzw. eines Kugelflächensegments hat. Die Lamellen können aber ebenso die Außenfläche eines flachen Kegels oder einer flachen Pyramide bilden. Im dargestellten Ruhezustand sind die Lamellen geschlossen, und die Schlitzflächen benachbarter Lamellen stoßen abdichtend gegeneinander an. Die Pfeile 5 stellen die Druckrichtung eines Fluids dar, wenn auf der Seite eines Beatmungsgeräts (nicht gezeigt) ein Überdruck herrscht, wie dies beim Einatmen des Patienten der Fall ist. Hier ist nur ein geringer Druck-Schwellwert erforderlich, um die Lamellen zum Öffnen des Ventils nach unten zu klappen. Beim Ausatmen (siehe auch Figur 2c) des Patienten wird auf der Patientenseite zunächst ein leichter Überdruck erzeugt, der gegen die nach unten zeigende konvexe Wölbung der Lamellen drückt und zu einem gegenseitigen Verpressen der Membranlamellen und damit zu einer Versperrung des Strömungskanals führt. Erst bei Überschreiten eines vorbestimmten Schwelldrucks zwischen etwa 5 und 15 mbar wird diese Sperrkraft überwunden, die Lamellen 3a-3d werden nach oben umgeklappt, und der Strömungskanal wird freigegeben. Wenn der Schwelldruck wieder unterschritten wird, klappen die Membranlamellen aufgrund ihrer eigenen Rückstellkräfte in ihre gewölbte Ausgangsstellung zurück, und der Strömungskanal ist wieder versperrt.

Figur 1c zeigt eine Querschnittsansicht durch Linie A-A aus Figur 1a, in der eine alternative Ausgestaltung der Lamellen 3a-3d zu sehen ist. Wie gezeigt, haben die Lamellen eine zur Mitte zunehmende Dicke, wodurch sich im Bereich der Schlitze 4a, 4b die Kontaktflächen zwischen benachbarten Membranlamellen in axialer Richtung erhöhen. Da in den Figuren die Schwenklinien 6 der Lamellen in axialer Richtung oberhalb der Mitte 7 der Kontaktfläche liegt, lassen sich die Lamellen mit leichtem Druck nach unten schwenken, wohingegen ein größerer Druck erforderlich ist, um die Lamellen nach oben zu schwenken.

Figur 1d zeigt eine alternative Ausgestaltung zu Figur 1c, bei der die Membranlamellen 3a-3b eine konstante Materialstärke haben, die größer ist als die Materialstärke des ringförmigen Haltebereichs 2. Auch hier liegt die Schwenklinie 6 der Lamellen in axialer Richtung oberhalb der Mitte 7 der Kontaktfläche zwischen den Lamellen. Dadurch lassen sich die Lamellen beim Einatmen mit leichtem Druck nach unten schwenken, wohingegen ein größerer Schwelldruck erforderlich ist, um die Lamellen nach oben zu schwenken.

Figur 1e zeigt eine Querschnittsansicht durch Linie A-A aus Figur 1a, in der eine alternative Ausgestaltung zu Figur 1d mit einer konstanten Materialstärke der gesamten Schlitzmembran 1 zu sehen ist. Zwischen dem Haltering 2 und dem mittleren Lamellenbereich 3 ist eine ringförmige Nut 8 vorgesehen. Die Funktion der unterschiedlichen Druck-Schwellwerte in verschiedenen Strömungsrichtungen ist auch in dieser Ausgestaltung realisiert.

Es ist offensichtlich, dass die Schlitzmembran 1 aus Figuren 1 a bis 1 e in ein vorzugsweise ringförmiges Ventilgehäuse montiert werden kann, wobei der Haltering 2 zum Beispiel in eine ringförmige Nut im Inneren des Ventilgehäuses eingesetzt werden kann. Auch andere Befestigungsarten sind denkbar, wie zum Beispiel Kleben, Verschweißen, Schmelzen, etc.

Figur 2a zeigt eine Ausgestaltung der erfindungsgemäßen Schlitzmembran aus Figur 1b im geschlossenen Zustand. In diesem Zustand herrscht auf beiden Seiten kein Druck, bzw. die Drücke auf beiden Seiten liegen jeweils unterhalb der Schwellwerte.

Figur 2b zeigt die Schlitzmembran aus Figur 2a in einem nach unten geöffneten Zustand, wobei der Druck in Richtung des Einatmens über einem geringen vorbestimmten Schwellwert von beispielsweise 0 bis 5 mbar liegt, der erforderlich ist, um die Membranlamellen 3a-3d nach unten zu drücken.

Figur 2c zeigt die Schlitzmembran aus Figur 2a in einem geöffneten Zustand, wobei der Druck in Richtung des Ausatmens wirkt. Dieser Druck liegt über dem Schwellwert von beispielsweise 10 oder 15 mbar, der erforderlich ist, um die Membranlamellen 3a-3d nach oben zu drücken.

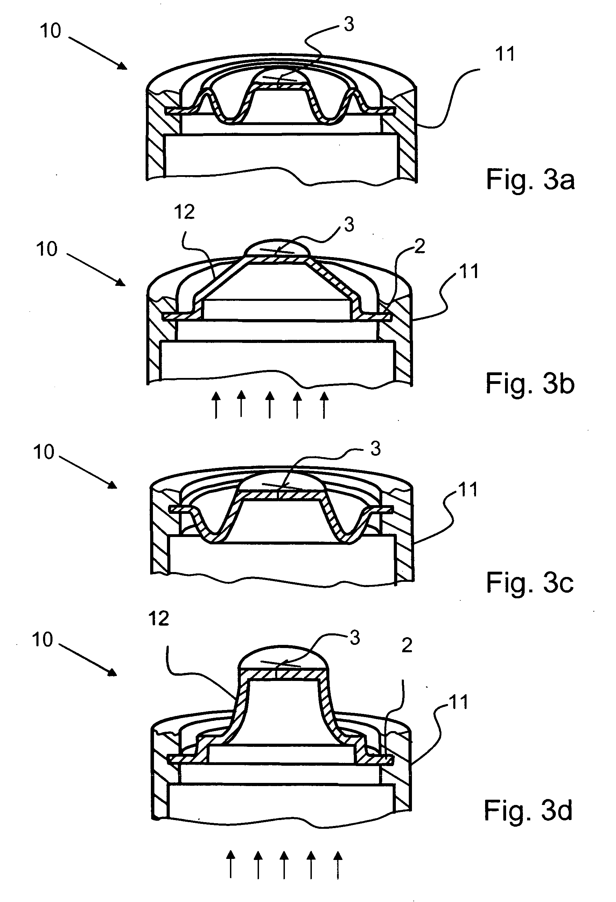

Figur 3a zeigt eine bevorzugte Ausgestaltung des Schlitzventils aus Figuren 1 und 2 im geschlossenen Zustand bzw. kurz vor Erreichen des geöffneten Zustands in Richtung des Einatmens (Überdruck auf der Seite des Beatmungsgeräts). Das Ventil 10 weist vorzugsweise ein im Wesentlichen rohrförmiges Ventilgehäuse 11 mit zwei allgemein gegenüberliegenden Anschlüssen auf und kann in zwei entgegengesetzten Richtungen mit Atemgas durchströmt werden. Das Ventilgehäuse 11 kann ausgestaltet sein, um einen Adapter oder einen Tubus anschließen zu können. Zwischen den beiden Anschlüssen ist eine Schlitzmembran 3 vorgesehen, die an ihrem ringförmigen Haltebereich 2 am Ventilgehäuse befestigt ist. Die Schlitzmembran 3 kann eine der Ausgestaltungen haben, wie sie in Figuren 1 und 2 gezeigt sind. Das in den Figuren 3a bis 3d gezeigte Ventil 10 ist als "Pop-Up" Ventil ausgestaltet. Zwischen dem inneren kreisförmigen Lamellenbereich 3 und dem äußeren Haltering 2 ist ein eindrückbarer bzw. faltbarer, ringförmiger Zwischenbereich 12 vorgesehen, der verschiedene Formen haben kann, wie in Figuren 3b und 3d am besten zu sehen ist. Wie in Figur 3b dargestellt, ist der Zwischenbereich 12 durch einen im Wesentlichen konisch zulaufenden Ringabschnitt gebildet, wohingegen der Zwischenbereich 12 in Figur 3d als ein zylindrischer Ringabschnitt gebildet ist, an den sich ein radial nach außen gekrümmter Wandabschnitt anschließt, der wiederum mit dem Haltering 2 verbunden ist. Figur 3a zeigt den geschlossenen Ruhezustand des Ventils bzw. den Zustand, der vorherrscht, wenn der Patient einatmet und nur ein geringer Schwellwert von beispielsweise weniger als 5 mbar (Überdruck auf der Seite des Beatmungsgeräts) zum Öffnen der Membranlamellen 3 erforderlich ist. Bei Überschreiten des Druck-Schwellwerts würden sich die Lamellen 3a-3d nach unten gerichtet öffnen, wie dies in Figur 2b dargestellt ist. Es ist offensichtlich, dass der Lamellenbereich 3 eine der in Figuren 1 b bis 1 e gezeigten Konfigurationen haben kann.

Der Zwischenbereich 12 kann, wie in Figuren 3a und 3c gezeigt, zusammen- bzw. ineinandergefaltet werden. In diesem Zustand sind die Membranlamellen 3a-3d geschlossen oder werden bei Überschreiten des Druck-Schwellwerts (Überdruck auf der Seite des Beatmungsgeräts = Einatmen des Patienten) nach unten geklappt. Wenn der Patient das Einatmen beendet, werden die Lamellen aufgrund ihrer Rückstellkraft wieder geschlossen. Wenn der Patient mit dem Ausatmen beginnt, werden die Lamellen zunächst gegeneinander verpresst, wodurch ein Umklappen der Lamellen verhindert wird. Ein Umklappen wird ferner dadurch verhindert, dass der zusammengefaltete Zwischenbereich 12 eine radial nach innen gerichtete Kraft auf die Lamellen aufbringt. Steigt der Druck auf der Patientenseite weiter an, wird der Zwischenbereich 12 zuerst entfaltet, wie in Figuren 3b und 3d gezeigt ist. In diesem Zustand sinkt die durch den Zwischenbereich auf die Lamellen wirkende Kraft, denn erst dann, wenn der Zwischenbereich entfaltet ist, erhält der Abschnitt zwischen den Membranlamellen und dem Zwischenbereich die nötige Flexibilität, um ein Umklappen der Membranlamellen in Richtung des Ausatmens zu ermöglichen. Folglich werden die Lamellen beim anschließenden Überschreiten des Druck-Schwellwerts (zum Beispiel 10 oder 15 mbar) auf der Patientenseite nach oben geklappt, wie in Figur 2c dargestellt ist. Wenn der Patient das Ausatmen wieder beendet, unterschreitet der Druck auf der Patientenseite wieder den obigen Schwellwert, und die Lamellen kehren aufgrund ihrer Rückstellkraft wieder in ihre geschlossene Ausgangsstellung zurück. Beim anschließenden Einatmen wird der Zwischenbereich wieder in die in Figuren 3a und 3c gezeigte Position zusammengefaltet. Somit wirkt der faltbare Zwischenbereich 12 als eine Art Sicherungseinrichtung gegen ein frühzeitiges Umklappen der Lamellen unterhalb eines definierten Druck-Schwellwerts.

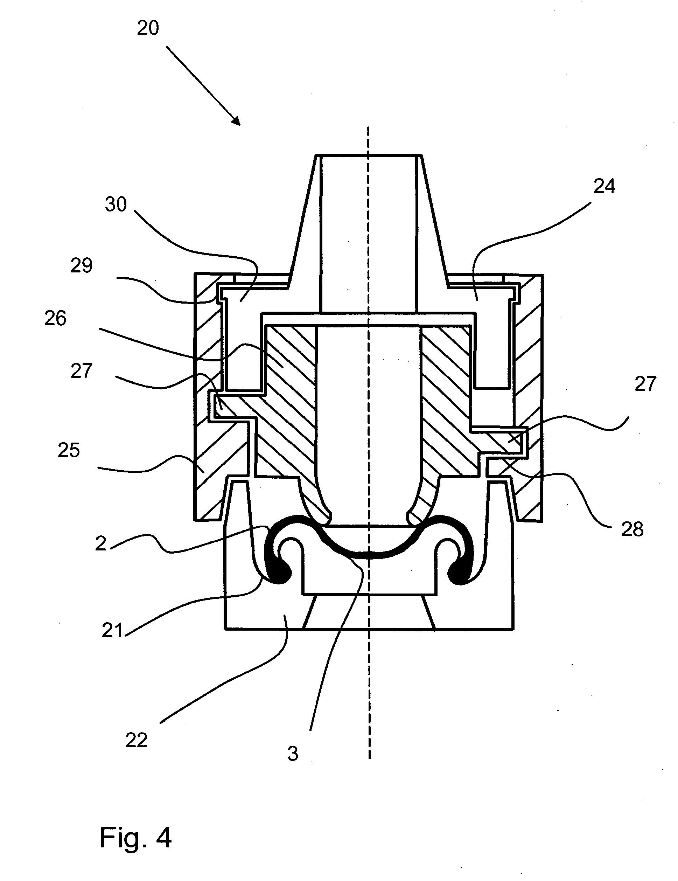

Figur 4 zeigt eine weitere Ausgestaltung der erfindungsgemäßen Schlitzventils, das zusätzlich mit einer Freigabeeinrichtung versehen ist. Das Schlitzventil 20 weist einen Lamellenbereich 3 auf, in dem eine Mehrzahl von geschlitzten Lamellen 3a-3d gebildet ist. Die Lamellen haben eine ähnliche Konfiguration wie in Figuren 1b und 2a-2c. Lediglich das Ausmaß der Wölbung der Lamellen ist bei der Ausgestaltung aus Figur 4 größer. Die Lamellen können aber auch die Konfiguration aus Figuren 1c-1e haben. Ferner ist der Haltering 2 bei der vorliegenden Ausgestaltung in Richtung der Wölbung C-förmig nach unten/innen gebogen und ausgestaltet, um in eine entsprechend geformte Aussparung 21 eines weiblichen Tubus-Konnektors 22 einzugreifen. Der weibliche Tubus-Konnektor 22 ist einstückig mit einem männlichen Tubus-Konnektor 24 verbunden, um das Ventilgehäuse zu bilden. Der männliche Tubus-Konnektor 24 ist ausgestaltet, um mit einer Öffnung eines Y-Stücks (nicht gezeigt) verbunden zu werden. In einer vorteilhaften Ausgestaltung ist zwischen Y-Stück und Schlitzventil eine geschlossene Absaugvorrichtung angebracht, deren Absaugekanüle die Schlitzmembran axial durchdringen kann zur Absaugung von Sekret. Neben dem aus dem weiblichen Konnektor 22 und dem männlichen Konnektor 24 gebildeten Gehäuse, der Schlitzmembran 1 mit beispielsweise vier oder sechs radial verlaufenden Schlitzen, weist das Ventil 20 außerdem einen drehbar am Gehäuse 22, 24 gehaltenen Drehring 25 und einen verschiebbar im Gehäuse gelagerten Offenhalter 26 auf. Der Offenhalter 26 kann so in den Wirkungsbereich der Schlitzmembran 1 bzw. des Lamellenbereichs 3 eingeschoben werden, dass die Membranlamellen 3a-3d der Schlitzmembran dauerhaft in Patientenrichtung geöffnet werden, um so im Inneren des Ventils die Strömungsmitte freizugeben und um keinen relevanten Strömungswiderstand darzustellen.

Der Offenhalter 26 verfügt über zwei Haltenasen 27, die durch das Gehäuse (zwischen dem männlichen und dem weiblichen Tubus-Konnektor 22, 24) hindurch in die schräge Bahn 28 des Drehrings 25 münden. Der Drehring 25 verfügt neben der schrägen Bahn 28 über eine ringförmige Nut 29, in die eine Wulst 30 des Gehäuses einrastet. Dadurch ist der Drehring 25 axial am Gehäuse fixiert. Bei Verdrehen des Drehrings wird der Offenhalter 26 über die schräge Bahn 28 axial verschoben und kann so wahlweise in eine Position nahe dem Y-Stück (Seite des Beatmungsgeräts) gebracht werden, wo der Offenhalter nicht mit den Membranlamellen eingreift und somit ein Druckabfall in der Lunge des Patienten unterhalb von beispielsweise 10 mbar vermieden wird. In der gegenüberliegenden, patientennahen Position greift der Offenhalter mit den Membranlamellen ein und drückt diese aus der Strömungsmitte heraus, wodurch ein bidirektionales Durchströmen von Fluid durch das Schlitzventil 20 ermöglicht wird.

## Patentansprüche

1. Schlitzventil in Kombination mit einem pneumatischen Schaltkreis eines Beatmungsgeräts, welches ausgestaltet ist, um bidirektional zu wirken und abhängig von der Strömungsrichtung eines Fluids bei verschiedenen Druck-Schwellwerten anzusprechen
**dadurch gekennzeichnet, dass** das Schlitzventil eine Membran (1) aufweist, wobei in der Mitte (3) der Membran (1) mehrere, sich kreuzende Schlitze (4a, 4b) vorgesehen sind, die mehrere Lamellen (3a-3d) bilden, wobei die Lamellen (3a-3d) so ausgestaltet sind, dass sie sich bei Aufbringung von Druck leichter in eine erste Richtung öffnen als in eine zweite Richtung, wobei dabei der Druck-Schwellwert zum Öffnen der Lamellen (3a-3d) in der ersten Richtung kleiner als in der zweiten Richtung ist.

2. Schlitzventil nach Anspruch 1, **dadurch gekennzeichnet, dass** das Schlitzventil selbstschließend ist und erst bei Überschreiten von Druck-Schwellwerten, die abhängig von der Strömungsrichtung eines Fluids verschieden sind, in den geöffneten Zustand übergeht, um das Durchströmen eines Fluids zu ermöglichen.

3. Schlitzventil nach einem der vorhergehenden Ansprüche, **dadurch gekennzeichnet, dass** der Schwelldruck in einer ersten Richtung in einem Bereich zwischen 0 mbar und 5 mbar liegt und in einer zweiten Richtung in einem Bereich zwischen 5 mbar und 15 mbar liegt.

4. Schlitzventil nach einem der vorhergehenden Ansprüche, **dadurch gekennzeichnet, dass** die Membran (1) an ihrem äußeren Rand durch einen Haltering (2) begrenzt ist und die Membran (1) vorzugsweise aus elastischem Kunststoff oder Gummi hergestellt ist.

5. Schlitzventil nach einem der vorhergehenden Ansprüche, **dadurch gekennzeichnet, dass** die Membran (1) eine runde, ovale, rechteckige oder quadratische Basisform hat.

6. Schlitzventil nach einem der vorhergehenden Ansprüche, **dadurch gekennzeichnet, dass** die Schlitzflächen benachbarter Lamellen (3a-3d) im geschlossenen Zustand abdichtend gegeneinander anstoßen.

7. Schlitzventil nach einem der vorhergehenden Ansprüche, **dadurch gekennzeichnet, dass** die Materialstärke der Lamellen (3a-3d) in einer axialen Richtung dicker ist als die Materialstärke des ringförmigen Randbereich, wobei vorzugsweise die Materialstärke der Lamellen (3a-3d) ausgehend von deren Schwenklinien in radialer Richtung auf die Mitte der Membran (1) zunimmt.

8. Schlitzventil nach einem der vorhergehenden Ansprüche, **dadurch gekennzeichnet, dass** der mit Schlitzen versehene Lamellenbereich (3) der Membran (1) eine gewölbte Fläche bildet, wobei die gewölbte Fläche vorzugsweise die Form einer Kuppel bzw. eines Kugelflächensegments hat, oder dass die gewölbte Fläche der Außenfläche eines flachen Kegels oder einer flachen Pyramide entspricht.

9. Schlitzventil nach einem der vorhergehenden Ansprüche, **dadurch gekennzeichnet, dass** die Lamellen (3a-3d) zumindest teilweise gewölbt sind.

10. Schlitzventil nach Anspruch 9, **dadurch gekennzeichnet, dass** die Lamellen (3a-3d) so gewölbt sind, dass im geschlossen Zustand die konvexe Seite der Wölbung in Richtung eines Patienten zeigt, wobei ein mäßiger Überdruck auf der konvexe Seite zu einer Verpressung der Wölbung entlang der Schlitze der Lamellen (3a-3d) und damit zu einer Versperrung des Strömungskanals führt.

11. Schlitzventil nach einem der vorhergehenden Ansprüche, **dadurch gekennzeichnet, dass** die Membran (1) an ihrem ringförmigen Rand (2) direkt oder indirekt in einem Gehäuse (11, 22, 24) fixiert ist.

12. Schlitzventil nach einem der vorhergehenden Ansprüche, **dadurch gekennzeichnet, dass** zwischen einem inneren kreisförmigen Schlitzbereich (3) der Membran (1) und dem äußeren Haltering (2) ein eindrückbarer bzw. faltbarer, ringförmiger Zwischenbereich (12) vorgesehen ist.

13. Schlitzventil nach einem der vorhergehenden Ansprüche, **dadurch gekennzeichnet, dass** das Schlitzventil eine zusätzliche Freigabeeinrichtung (26) aufweist, die bei Betätigung die Lamellen (3a-3d) aus der geschlossenen Position herausklappt, so dass der Strömungskanal im Inneren des Ventils freigegeben ist.

14. Schlitzventil nach Anspruch 13, **dadurch gekennzeichnet, dass** das Ventil einen Drehring (25) und einen Offenhalter (26) aufweist, wobei der Offenhalter (26) so in den Wirkungsbereich der Membran (1) eingeschoben wird, dass die Lamellen (3a-3d) der Membran (1) dauerhaft die Strömungsmitte freigeben und keinen relevanten Strömungswiderstand darstellen, wobei der Offenhalter (26) vorzugsweise über zwei Haltenasen (27) verfügt, die durch das Gehäuse (22, 24) hindurch in eine schräge Bahn (28) des Drehrings (25) münden, wodurch der Offenhalter (26) bei Verdrehen des Drehrings (25) axial verschoben wird.

15. Schlitzventil nach Anspruch 14, **dadurch gekennzeichnet, dass** der Drehring (25) eine ringförmige Nut (29) aufweist, in die eine Wulst (30) des Gehäuses (22, 24) einrastet, wodurch der Drehring gegen axiale Verlagerung am Gehäuse (22, 24) fixiert ist.

## Claims

1. Slit valve in combination with a pneumatic switch circuit of a respiration apparatus, wherein the slit valve is formed in order to act bi-directionally and to respond depending on the flow direction of a fluid with different pressure thresholds, **characterised in that** the slit valve has a membrane (1), wherein several slits (4a, 4b) crossing each other are provided in the middle (3) of the membrane (1) which form several lamellae (3a - 3d), wherein the lamellae (3a - 3d) are formed such that with application of pressure they open easier in a first direction than in a second direction, wherein in the course of this the pressure threshold for opening the lamellae (3a - 3d) in a first direction is smaller than in the second direction.

2. Slit valve according to claim 1, **characterised in that** the slit valve is self-closing and only passes into the open state when crossing pressure thresholds which in dependency of the flow direction of a fluid differ in order to allow the fluid to pass.

3. Slit valve according to one of the preceding claims, **characterised in that** the expansion pressure in a first direction is in a range between 0. mbar and 5 mbar and in a second direction is in a range between 5 mbar and 15 mbar.

4. Slit valve according to one of the preceding claims, **characterised in that** the membrane (1) is limited by a retaining ring (2) at its outer edge and **in that** the membrane (1) is preferably made of elastic plastic or rubber.

5. Slit valve according to one of the preceding claims, **characterised in that** the membrane (1) has a round, oval, rectangular or square basic form.

6. Slit valve according to one of the preceding claims, **characterised in that** the slit areas of adjacent lamellae (3a - 3d) trigger sealingly against each other in the closed state.

7. Slit valve according to one of the preceding claims, **characterised in that** the thickness of the lamellae (3a - 3d) in the axial direction is thicker than the thickness of the annular edge region, wherein the thickness of the lamellae (3a - 3d) in the radial direction preferably increases from their pivot lines to the middle of the membrane (1).

8. Slit valve according to one of the preceding claims, **characterised in that** the lamellae region (3) bearing slits of the membrane (1) forms a curved surface, wherein the curved surface preferably has the form of a dome or a spherical segment respectively, or **in that** the curved surface corresponds to the outer surface of a flat taper or a flat pyramid.

9. Slit valve according to one of the preceding claims, **characterised in that** the lamellae (3a - 3d) are at least partly curved.

10. Slit valve according to claim 9, **characterised in that** the lamellae (3a - 3d) are curved such that the convex side of the curvature in the closed state points in the direction of a patient, wherein a moderate pressure on the convex side leads to an injection of the curvature along the slits of the lamellae (3a - 3d) and therefore to a blockage of the flow channel.

11. Slit valve according to one of the preceding claims, **characterised in that** the membrane (1) at its annular edge (2) is fixed directly or indirectly in a housing (11, 22, 24).

12. Slit valve according to one of the preceding claims, **characterised in that** a crushable or foldable annular intermediate space (12) is provided between an inner circular slit region (3) of the membrane (1) and the outer retaining ring (2).

13. Slit valve according to one of the preceding claims, **characterised in that** the slit valve has an additional release device (26), which when actuated folds out the lamellae (3a - 3d) out of a closed position, so that the flow channel inside the valve is released.

14. Slit valve according to claim 13, **characterised in that** the valve has a rotating ring (25) and an open keeper (26), wherein the open keeper (26) is inserted into the affected area of the membrane (1) such that the lamellae (3a - 3d) of the membrane (1) release the flow centre permanently and represent no relevant flow resistance, wherein the open keeper (26) has preferably two retaining (27), which result in an oblique path (28) of the rotating ring (25) through the housing (22, 24), wherein the open keeper (26) is moved axially in rotating the rotating ring (25).

15. Slit valve according to claim 14, **characterised in that** the rotating ring (25) has an annular groove (29), into which a bead (30) of the housing (22, 24) engages, whereby the rotating ring is fixed against axial displacement on the housing (22, 24).

## Revendications

1. Soupape à fentes en combinaison avec un circuit pneumatique d'un respirateur artificiel, qui est conçue pour avoir une action bidirectionnelle et réagir en présence de différentes valeurs seuils de pression, en fonction du sens d'écoulement d'un fluide,
**caractérisée par le fait que** la soupape à fentes présente une membrane (1), la membrane (1) comportant au milieu (3) plusieurs fentes (4a, 4b) qui se croisent et forment plusieurs lamelles (3a-3d), lesdites lamelles (3a-3d) étant agencées de manière telle que, lors de l'application d'une pression, elles s'ouvrent plus facilement dans un premier sens que dans un deuxième sens, la valeur seuil de pression pour l'ouverture des lamelles (3a-3d) étant plus petite dans le premier sens que dans le deuxième sens.

2. Soupape à fentes selon la revendication 1, **caractérisée par le fait que** la soupape à fentes est à fermeture automatique et ne passe à l'état ouvert pour permettre le passage d'un fluide qu'en cas de dépassement de valeurs seuils de pression qui varient en fonction du sens d'écoulement d'un fluide.

3. Soupape à fentes selon une des revendications précédentes, **caractérisée par le fait que** la pression de seuil dans un premier sens est comprise dans une plage entre 0 mbar et 5 mbars et dans un deuxième sens dans une plage entre 5 mbars et 15 mbars.

4. Soupape à fentes selon une des revendications précédentes, **caractérisée par le fait que** la membrane (1) est délimitée sur son bord extérieur par une bague de support (2), et la membrane (1) est réalisée de préférence à partir de matière plastique élastique ou de caoutchouc.

5. Soupape à fentes selon une des revendications précédentes, **caractérisée par le fait que** la membrane (1) a une forme de base ronde, ovale, rectangulaire ou carrée.

6. Soupape à fentes selon une des revendications précédentes, **caractérisée par le fait qu'**à l'état fermé, les surfaces de fente de lamelles (3a-3d) voisines sont en contact étanche les unes avec les autres.

7. Soupape à fentes selon une des revendications précédentes, **caractérisée par le fait que** l'épaisseur de matière des lamelles (3a-3d) est plus grande dans un sens axial que l'épaisseur de matière de la zone de bord annulaire, l'épaisseur de matière des lamelles (3a-3d) augmentant de préférence à partir de leurs lignes d'oscillation et radialement en direction du milieu de la membrane (1).

8. Soupape à fentes selon une des revendications précédentes, **caractérisée par le fait que** la zone lamellaire (3), dotée de fentes, de la membrane (1) constitue une surface bombée, laquelle a de préférence la forme d'une calotte ou d'un segment de surface sphérique, ou **par le fait que** la surface bombée correspond à la surface extérieure d'un cône plat ou d'une pyramide plate.

9. Soupape à fentes selon une des revendications précédentes, **caractérisée par le fait que** les lamelles (3a-3d) sont au moins partiellement bombées.

10. Soupape à fentes selon la revendication 9, **caractérisée par le fait que** les lamelles (3a-3d) sont incurvées de manière à ce que, à l'état fermé, la face convexe du bombement soit orientée en direction d'un patient, une surpression modérée sur la face convexe entraînant une compression du bombement le long des fentes des lamelles (3a-3d) et donc l'obturation du canal d'écoulement.

11. Soupape à fentes selon une des revendications précédentes, **caractérisée par le fait que** la membrane (1) est fixée par son bord (2) annulaire directement ou indirectement dans un boîtier (11, 22, 24).

12. Soupape à fentes selon une des revendications précédentes, **caractérisée par le fait qu'**entre une zone fendue (3) circulaire intérieure de la membrane (1) et la bague de support (2) extérieure, il est prévu une zone intermédiaire (12) annulaire pouvant être enfoncée ou pliée.

13. Soupape à fentes selon une des revendications précédentes, **caractérisée par le fait que** la soupape à fentes présente un dispositif de libération (26) supplémentaire qui, lors de l'actionnement, rabat les lamelles (3a-3d) pour leur faire quitter leur position fermée, de sorte que le canal d'écoulement à l'intérieur de la soupape est libéré.

14. Soupape à fentes selon la revendication 13, **caractérisée par le fait que** la soupape présente une bague rotative (25) et un élément de maintien d'ouverture (26), ledit élément de maintien d'ouverture (26) étant glissé dans la zone d'action de la membrane (1) de manière telle que les lamelles (3a-3d) de la membrane (1) libèrent de façon durable le centre d'écoulement et ne représentent pas de résistance hydraulique importante, l'élément de maintien d'ouverture (26) comportant de préférence deux ergots de maintien (27) qui traversent le boîtier (22, 24) et débouchent dans un chemin (28) oblique de la bague rotative (25), grâce à quoi l'élément de maintien d'ouverture (26) est déplacé axialement lors de la rotation de la bague rotative (25).

15. Soupape à fentes selon la revendication 14, **caractérisée par le fait que** la bague rotative (25) présente une rainure (29) annulaire, dans laquelle s'enclenche un bourrelet (30) du boîtier (22, 24), grâce à quoi la bague rotative est immobilisée en déplacement axial sur le boîtier (22, 24).
